(19) Europäisches Patentamt
European Patent Office
Office européen des brevets

(11) **EP 2 389 971 A1**

(12) **EUROPEAN PATENT APPLICATION**
published in accordance with Art. 153(4) EPC

(43) Date of publication:
**30.11.2011 Bulletin 2011/48**

(51) Int Cl.:
***A61M 16/00*** $^{(2006.01)}$

(21) Application number: **10811442.2**

(86) International application number:
**PCT/JP2010/004858**

(22) Date of filing: **02.08.2010**

(87) International publication number:
**WO 2011/024383 (03.03.2011 Gazette 2011/09)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.08.2009 JP 2009197889**

(71) Applicants:
• **Ulvac Kiko, Inc.**
  **Saito-shi, Miyazaki 881-0037 (JP)**
• **National University Corporation Tokyo Medical and Dental University Bunkyo-ku Tokyo 113-8510 (JP)**

(72) Inventors:
• **AIKAWA, Junichi**
  **Saito-shi**
  **Miyazaki 881-0037 (JP)**
• **WAKAMATSU, Hidetoshi**
  **Tokyo 113-8510 (JP)**
• **UTSUKI, Tomohiko**
  **Tokyo 113-8510 (JP)**

(74) Representative: **Schmid, Wolfgang**
  **Lorenz & Kollegen**
  **Patentanwälte Partnerschaftsgesellschaft**
  **Alte Ulmer Strasse 2**
  **89522 Heidenheim (DE)**

(54) **VENTILATOR AND OPERATION METHOD THEREFOR**

(57)     [Object] To provide a compact and low-power-consumption artificial respirator and an operation method therefor.

[Solving Means] In an artificial respirator 100 according to an embodiment of the present invention, the control section 17 controls the valve mechanism 5, 8 to be driven, to thereby alternately switch between a first state for feeding inspiratory air stored in the reservoir tank 4 to a patient and a second state for releasing expired air of the patient. The pressurizing pump 1 has a discharge function for reversing a reduction of pressure within the reservoir tank 4, which is caused due to the inspiration, within an expiration period of time. Here, the expiration period of time (second period of time) is set to be equal to or longer than the inspiration period of time (first period of time), and hence a pressurizing pump having a small volume is allowed to supply needed inspiratory air into the reservoir tank 4. Thus, it is possible to reduce the volumes of the pressurizing pump 1 and the reservoir tank 4, and at the same time to achieve a reduction of driving electric power for the pressurizing pump 1.

FIG.6

**Description**

Technical Field

The present invention relates to an artificial respirator that supplies inspiratory air (inspiratory gas) when artificial respiration is performed on a user (patient) in a resuscitation procedure, and an operation method therefore.

Background Art

In recent years, automatic external defibrillators (AED) have been permitted to be used by ordinary people other than the healthcare professionals such as doctors, emergency life saving technicians, nurses, and the like, and the AEDs have been widely provided at places where many people gather, such as airports, stations, and buildings, which contribute to increase the survival rate. In the resuscitation, cardiac arrest and respiration cease have to be handled. Although the AED is effective with respect to the cardiac arrest, for handling the respiration cease, artificial respiration (mouth to mouth, for example) is needed at the present time. Thus, if compact artificial respirators that are easy to be transported for first-aid are widely distributed together with the AEDs, it is possible to further increase the survival rate.

The commonly distributed artificial respirators are manufactured on the assumption that they are used by the healthcare professionals, and setting of the respiratory pattern, the inspiratory pressure, concentration of oxygen, concentration of $CO_2$ in expiration, and the like are adapted to be set or to be automatically set depending on patients, and hence the artificial respirators are large apparatuses. In addition, in order to supply the inspiratory gas, the infrastructure of hospital is used, or most artificial respirators use a compressed air container or a compressed oxygen container even if the artificial respirators are transportable. Therefore, the artificial respirators cannot be easily transported for use.

On the assumption that the artificial respirator is provided together with the AED, it is important to reduce the size and the weight of the apparatus. As a method of supplying the inspiratory gas, there is a method using a pressure vessel. In this case, assumed that one-time inspiratory volume is 0.5 L, the respiratory rate is 12 times per minute, and the procedure period of time is 60 minutes, necessary air amount is 360 L. Assumed that the gas pressure is 10 Mpa, the gas volume is 3.6 L. When the pressure vessel available in the market is used, the pressure vessel has a volume of 10 L, and thus it has a weight of about 12 kg. Therefore, it cannot be easily transported.

On the other hand, Patent Document 1 below discloses a portable artificial respirator. In this artificial respirator, the inspiratory gas is generated by a dual head air compressor. For switching of respiration, an air driven valve is employed. In order to generate compressed air for driving this valve, a single head air compressor is incorporated. Those two air compressors are alternately operated, to thereby form a respiratory pattern.

[Cited Document]

[Patent Document]

Patent Document 1: Japanese Unexamined Patent Application Publication No. 2007-525273 (paragraphs [0036] to [0039])

Disclosure of the Invention

Problem to be solved by the Invention

In recent years, similarly to the AEDs, distribution of compact artificial respirators easy to be transported for first-aid has been strongly demanded. In order to respond to such a demand, there is a need for overcoming various technical problems such as a reduction of the size and the weight enough to easily transport the apparatus from its setting place and a reduction of the electric power consumption. However, with the artificial respirator disclosed in Patent Document 1 above, such problems cannot be sufficiently overcome.

In view of the above-mentioned circumstances, it is an object of the present invention to provide a compact and low-power-consumption artificial respirator and an operation method therefor.

Means for solving the Problem

In order to achieve the above-mentioned object, an artificial respirator according to an embodiment of the present invention includes a mask, a reservoir tank, a valve mechanism, a control section, and a pressurizing pump. The mask is to be worn by a patient. The reservoir tank stores inspiratory air. The valve mechanism is connected between the mask and the reservoir tank, and has a first state and a second state. In the first state, the inspiratory air contained in the reservoir tank is fed to the mask. In the second state, expired air of the patient, which is discharged from the mask, is released. The control section is capable of alternately switching between the first state and the second state. The control section controls the valve mechanism to be driven so that the first state is kept for a first period of time and the second state is kept for a second period of time equal to or longer than the first period of time. The pressurizing pump is connected to the reservoir tank to supply the inspiratory air, which is consumed in the first period of time, within the second period of time.

Further, in order to achieve the above-mentioned object, an operation method for an artificial respirator according to an embodiment of the present invention includes a step of feeding inspiratory air contained in a reservoir tank to a mask, that a patient wears, for a first period of time. Expired gas of the patient is released under a state of blocking a communication between the reservoir tank and the mask for a second period of time equal to or longer than the first period of time. By a pressurizing pump connected to the reservoir tank, the inspiratory air, which is consumed in the first period of time, is supplied within the second period of time. The inspiratory air contained in the reservoir tank is fed to the mask for the first period of time.

Brief Description of Drawings

[Fig. 1] A view showing a respiratory pattern of an artificial respirator according to an embodiment of the present invention.
[Fig. 2] A piping diagram of a gas supply system in a case of directly feeding inspiratory gas from a pressurizing pump to a mask.
[Fig. 3] A piping diagram of the gas supply system in a case of feeding the inspiratory gas from the pressurizing pump to the mask through a reservoir tank.
[Fig. 4] A view showing a change over time of a gas amount within the reservoir tank in the embodiment of the present invention.
[Fig. 5] A view showing a change over time of a pressure within the reservoir tank in the embodiment of the present invention.
[Fig. 6] A piping diagram showing an entire configuration of the artificial respirator according to the embodiment of the present invention.
[Fig. 7] A view showing the performance of the pressurizing pump to be used in the embodiment of the present invention, which shows a relation between a discharging flow rate and a pressure when the rotational speed is varied.
[Fig. 8] A view showing an example of the electric power consumption of the artificial respirator according to the embodiment of the present invention.

Best Mode(s) for Carrying Out the Invention

An artificial respirator according to an embodiment of the present invention includes a mask, a reservoir tank, a valve mechanism, a control section, and a pressurizing pump.

The mask is worn by the patient so as to cover the mouth and the nose, for example. The control section controls the valve mechanism to be driven, to thereby alternately switch between a first state for feeding inspiratory air stored in the reservoir tank to the patient and a second state for releasing expired air of the patient. The pressurizing pump has a discharge function for reversing a reduction of pressure within the reservoir tank, which is caused due to the inspiration, for an expiration period of time. Here, the expiration period of time (second period of time) is set to be equal to or longer than the inspiration period of time (first period of time), and hence the pressurizing pump having a small volume is allowed to supply needed inspiratory air into the reservoir tank. Thus, it is possible to reduce the volumes of the pressurizing pump and the reservoir tank, and at the same time to achieve a reduction of driving electric power for the pressurizing pump. Therefore, according to the artificial respirator, it is possible to achieve a reduction of the size and the electric power consumption.

The artificial respirator may further include a battery and a casing. The battery supplies electric power to the valve mechanism, the control section, and the pressurizing pump. The casing houses the battery, the mask, the reservoir tank, the valve mechanism, the control section, and the pressurizing pump.
Thus, it becomes possible to easily perform installation of the artificial respirator in place and transportation of the artificial respirator from such a place.

The artificial respirator may further include a guiding means for providing audio or video guidance for an operating procedure for the artificial respirator.
In this manner, ordinary people other than the healthcare professionals are allowed to operate the artificial respirator.

An operation method for an artificial respirator according to an embodiment of the present invention includes feeding inspiratory air contained within a reservoir tank to a mask, that a patient wears, for a first period of time. The inspiratory air, which is consumed in the first period of time, is supplied within a second period of time equal to or longer than the first period of time by a pressurizing pump connected to the reservoir tank.

According to the operation method for the artificial respirator, it is possible to reduce the volumes of the pressurizing pump and the reservoir tank, and at the same time to achieve a reduction of driving electric power for the pressurizing pump. Thus, it is possible to achieve a reduction of the size and the electric power consumption of the artificial respirator.

Further, the pressurizing pump is continuously operated for the first period of time and the second period of time at a constant rotational speed, and hence it is possible to reduce the electric power consumption as compared to a case of operating the pressurizing pump in an intermittent manner.

Hereinafter, with reference to the drawings, an embodiment of the present invention will be described. Although in the following description, specific integers are described, it is needless to say that the present invention is not limited to those integers.

The artificial respirator according to the embodiment of the present invention uses a pressurizing pump having a relatively small volume, to thereby realize a reduction in size and weight of the apparatus. In the case of realizing the artificial respirator that anyone can use for first-aid, it is so difficult to optimize the setting of the inspiratory volume (ventilatory volume), the respiratory rate, the ratio of expiration and inspiration, and the inspiratory pressure depending on users (patients). In view of this, the respiratory pattern is set to one pattern for safety reasons. It should be noted that the inspiratory volume can be selected for child or adult. When the airway pressure exceeds 40 cmH$_2$O, problems may occur in the trachea or the lung, and hence the inspiratory pressure is typically set to range from 10 to 30 cmH$_2$O, and in this embodiment to 30 cmH$_2$O. The respiratory rate is set to 12 times per minute, the ratio of the inspiration period of time and the expiration period of time is set to 1:2, and the inspiratory volume (ventilatory volume) is set to 0.5 L.

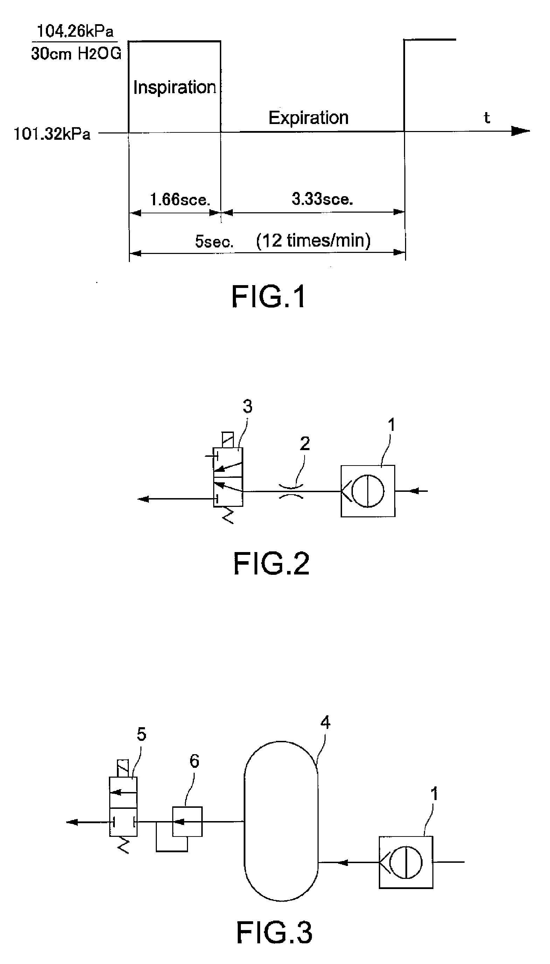

Fig. 1 shows the respiratory pattern. It should be noted that the pressure (vertical axis) is an absolute pressure, and is expressed in Pascal (Pa). Further, in the following discussion, the flow rate is expressed in NL/min with 0°C, 1 atmosphere being as a reference. The respiratory rate is 12 times per minute and the ratio of the inspiration period of time and the expiration period of time is 1:2, and thus, the respiratory interval is 5 seconds, the inspiration period of time is 1.66 seconds, and the expiration period of time is 3.33 seconds.

Fig. 2 shows a case where the pressurizing pump 1 is constantly operated and the switching of respiration is performed through a flow rate control orifice 2 by a solenoid valve 3. The ventilatory volume is 0.5 L at 20°C and the pressure is 30 cmH$_2$O = 104.264 kPa, and thus, the gas amount G1 consumed in one inspiration can be calculated by the following expression:

```
G1 = ventilatory volume × pressure = 0.5 × 104.26

= 52.13 kPa*L ...(1).
```

This ventilation is performed in 1.66 seconds, and thus, the gas flow rate Q1 can be calculated by the following expression:

```
Q1 = (G1/1.66) × 60

= (52.13/1.66) × 60 = 1884 kPa*L/min ...(2).
```

When reducing into 1 atmosphere, 20°C,

```
1884/101.3 = 18.6 L/min ...(3)
```

is established. When setting to the normal state of 0°C, 1 atmosphere,

```
18.6×(273/293) = 17.33 NL/min ...(4).
```

The electric power consumption of the pressurizing pump, which can satisfy this flow rate, ranges from approximately 30 W to 50 W, although it varies in a large degree depending on the structure of the pump and the efficiency of the drive motor. Thus, it is necessary to prepare a large battery for driving.

In order to solve such a problem, there can be conceived an operation method in which the pressurizing pump is stopped for the expiration period of time. The pressurizing pump can be stopped by 2/3 of respiratory period of time, and hence the capacity of the battery can be reduced. It should be noted that the following problems occur: during activation of the pressurizing pump, large activation electric current is needed, and hence the battery capable of applying large electric current for a short time is needed; and the typical pressurizing pump is manufactured on the assumption of continuous operation, and hence the mechanical duration of life is shorten due to the repeated activations.

In view of this, in this embodiment, the above-mentioned problems are overcome in the following manner. That is, in the respiratory pattern of Fig. 1, in the respiratory interval of 5 seconds, the inspiration period of time is 1.66 seconds and the expiration period of time is 3.33 seconds longer than the inspiration period of time. Thus, it may took 5 seconds to prepare the inspiratory gas. Then, a method of using a gas storage (reservoir tank) 4 shown in Fig. 3 to store the inspiratory gas in the reservoir tank within 3.33 seconds of the expiration period of time is considered. In this case, the pressurizing pump is continuously operated.

Assumed that the discharged gas amount demanded to the pressurizing pump is Q2 (KPa*L/min), the following expression is established:

$$G1 = (5/60) \times Q2 \ldots (5).$$

According to Expression (1),

$$Q2 = 625.56 \text{ kPa* L/min} \ldots (6).$$

When reducing into 20°C, 1 atmosphere,

$$625.56/101.3 = 6.17 \text{ L/min} \ldots (7).$$

When setting this to the normal state of 0°C, 1 atmosphere,

$$6.17 \times (273/293) = 5.75 \text{ NL/min} \ldots (8).$$

According to the above-mentioned calculation, when the reservoir tank is used, as compared to the case without reservoir tank, according to Expression (4), downsizing from the pressurizing pump of 17.33 NL/min to the pressurizing pump of 5.75 NL/min can be achieved.

Next, the volume of the reservoir tank 4 is determined. With reference to Fig. 3, to the outlet side of the reservoir tank 4, a pressure reducing valve 6 is connected, and to the outlet side of the pressure reducing valve 6, a solenoid valve 5 that communicates to a mask (not shown) is connected. The solenoid valve 5 is switched between a communication state and a blocking state during respiration.

In the example of Fig. 3, regarding the volume of the reservoir tank 4, the inspiratory gas is supplied through the pressure reducing valve 6 to the mask, and hence, after consummation of the inspiratory gas, the pressure enough to stably operate the pressure reducing valve 6 has to be remained. Here, the inspiratory pressure of 104.26 kPa (30 cmH$_2$O) is set as the minimum pressure. Assumed that the gas amount within the reservoir tank 4 is G (KPa*L), the gas contained in the reservoir tank is consumed as the inspiratory gas while the gas is being supplied from the pressurizing pump 1, and hence the gas amount within the reservoir tank at the termination of the inspiration can be expressed as follows:

$$G-G1+Q2 \times (1.66/60) \ldots (9).$$

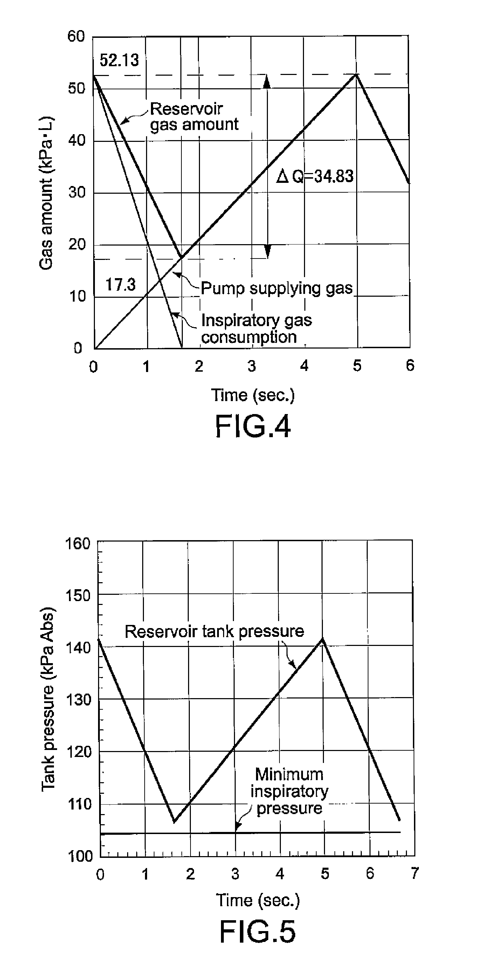

It should be noted that the gas amount at the termination of the expiration is one that is obtained by adding $Q2 \times (3.33/60)$ to the gas amount at the termination of the inspiration. Thus, the gas amount reduction ($\Delta Q$) of the reservoir tank at the termination of the inspiration is, according to Expression (9), 34.83 KPa*L. In Fig. 4, the gas amount change of the reservoir tank with respect to period of time is shown.

Assumed that the tank volume is V, the pressure of the reservoir tank 4 is expressed by the following expression:

$$P = G/V \ldots(10).$$

Here, the pressure change on the assumption that the reservoir tank volume is 1 L and the initial pressure is 141.3 kPa (40 kPaG) is shown in Fig. 5. The pressure change due to consummation of the inspiratory gas is as follows:

$$\Delta P = G/V = 34.83/1 = 34.83 \text{ kPa,}$$

the pressure at the termination of the inspiration is 141.26 - 34.83 = 106.43 kPa, and the minimum inspiratory pressure of 104.26 kPa can be satisfied.

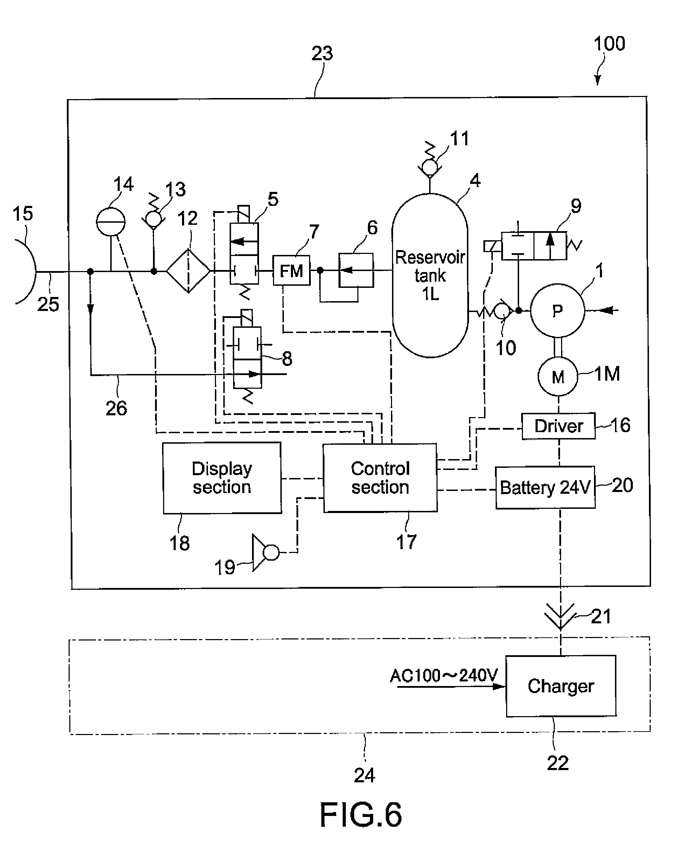

Fig. 6 is a schematic view showing the whole of the artificial respirator according to the embodiment of the present invention.

The artificial respirator 100 of this embodiment includes a casing 23 that houses a gas supply system, a control system, a power supply system, and the like. The casing 23 is detachably attached to a base 24 placed in place in doors or out of doors, and is detached from the base 24 and transported to a using place during use. The size of the casing 23 is, for example, about 35 cm in length, width, and height, respectively.

The base 24 is provided with a charger 22 connected to a power supply source such as a commercial power supply. The casing 23 has a built-in battery 20 constituting the power supply system, and is capable of charging the battery 20 through a conductor 21 when placed in the base 24. As the battery 20, for example, a nickel-hydrogen battery (24 V) having a capacity of 3 AH can be used.

The gas supply system of the artificial respirator 100 includes the pressurizing pump 1, the reservoir tank 4, the pressure reducing valve 6, an inspiratory solenoid valve 5, an expiratory solenoid valve 8, and the mask 15. The pressurizing pump 1 is connected to the reservoir tank 4, and supplies inspiratory gas (inspiratory air) generated by compressing the air into the reservoir tank 4. As the pressurizing pump 1, for example, a diaphragm pomp is used. The pressurizing pump 1 is driven by a DC brushless motor 1M. The motor 1M is provided with driving electric power through a driver 16 from the battery 20, and changes output waveform according to an instruction from a control section 17 to thereby operate the pressurizing pump 1 at an arbitrary rotational speed.

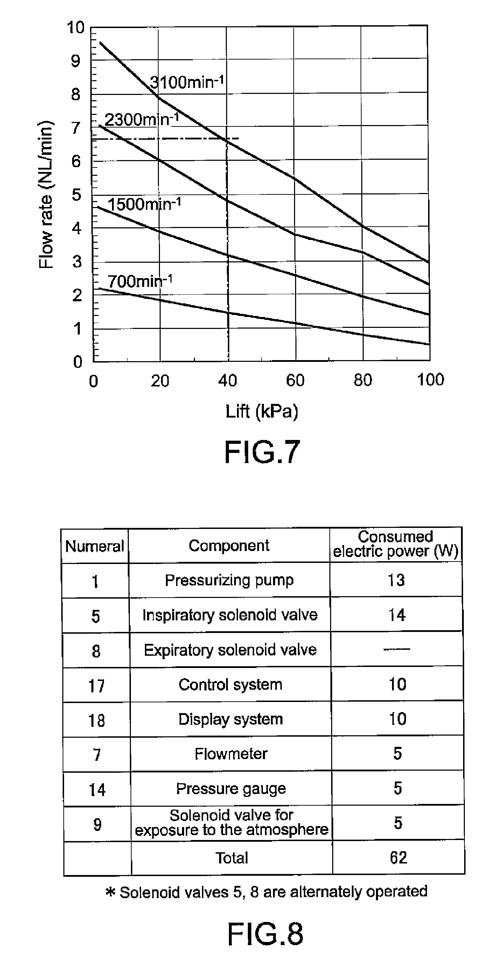

A relation between a flow rate and a discharging pressure (lift) of the pressurizing pump is shown in Fig. 7. As mentioned above, it is sufficient that the pressurizing pump 1 have a performance of 5.75 NL/min at the pressure of 141.3 kPa (the pressure with the atmosphere being as a reference is 40 kPaG) (Expression (8)). The pressurizing pump (manufactured by ULVAC KIKO Inc. (trade name "DFC-4-2")) having characteristics shown in Fig. 7 has a discharge flow rate of 5.75 NL/min at a rotational speed of 3100 min$^{-1}$ (rpm) at the pressure of 40 kPa, which can achieve the object sufficiently. Under this operating condition, the electric power consumption was 13 W.

The pressurizing pump 1 is stopped for a significantly long time, and hence in order to immediately supply the inspiratory gas, the inspiratory gas contained in the reservoir tank 4 is stored while being pressurized. Typically, the diaphragm pump includes an inspiratory valve and an expiratory valve, and hence by stopping the reservoir tank while being pressurized, a discharge valve is pressed due to the pressure and fixed, with a result that during activation, it is impossible to generate the inspiratory gas. In view of this, in this embodiment, between the pressurizing pump 1 and the reservoir tank 4, there is provided a check valve 10 for preventing the counter flow of the inspiratory gas from the reservoir tank 4 to the pressurizing pump 1. Further, a solenoid valve 9 for exposure to the atmosphere is attached between the pressurizing pump 1 and the check valve 10, which releases the pressure during stop of the pump to prevent the pressure of the inspiratory gas from being applied to the discharge valve. The solenoid valve 9 for exposure to the atmosphere is driven by the control section 17.

The reservoir tank 4 includes a relief valve 11. The volume of the reservoir tank 4 is for example 1 L, and when the inner pressure of the reservoir tank 4 becomes equal to or higher than 40 kPaG, the relief valve 11 is opened. In this manner, the pressurizing pump 1 is prevented from being excessively loaded.

To the downstream side of the reservoir tank 4, the pressure reducing valve 6 is attached, and thus, the downstream side of the pressure reducing valve 6 is kept at 104.26 kPa (3 KPaG). To the downstream side of the pressure reducing valve 6, the inspiratory solenoid valve 5 is connected through a flowmeter 7. The inspiratory solenoid valve 5 functions as a supply valve for feeding the inspiratory gas contained in the reservoir tank 4 to the mask 15. The inspiratory solenoid valve 5 has two positions of a communication position and a blocking position, and is switched to the communication position during inspiration, and to the blocking position during expiration. The switching of the inspiratory solenoid valve 5 is controlled by the control section 17. The inspiratory gas, which has passed through the inspiratory solenoid valve 5, is fed through a first conduit 25 to the mask 15.

The first conduit 25 links between the inspiratory solenoid valve 5 and the mask 15, and a part of the first conduit 25 may be made of a flexible piping material. The first conduit 25 is housed in the casing 23 when not used, and is put out from the casing 23 together with the mask 15 when used. To the first conduit 25, a filter 12, a relief valve 13, and a pressure gauge 14 are attached. The relief valve 13 is opened when the first conduit 25 reaches a predetermined abnormal pressure value, for example, when the airway of the patient is closed.

Further, to the first conduit 25, a second conduit 26 that communicates to the expiratory solenoid valve 8. The expiratory solenoid valve 8 has two positions of a communication position and a blocking position, and is switched to the blocking position during inspiration, and to the communication position during expiration. The switching of the expiratory solenoid valve 8 is controlled by the control section 17.

The control section 17 constitutes the control system of the artificial respirator 100. The control section 17 is configured by a computer, and controls the pressurizing pump 1 (driver 16), the inspiratory solenoid valve 5, the expiratory solenoid valve 8, and the solenoid valve 9 for exposure to the atmosphere to be driven. Here, the inspiratory solenoid valve 5 and the expiratory solenoid valve 8 constitute a "valve mechanism" according to the present invention. The control section 17 controls the switching of the inspiratory solenoid valve 5 and the expiratory solenoid valve 8 in synchronization, to thereby alternately switch a state (first state) of feeding the inspiratory gas to the patient and a state (second state) of discharging the expired air from the patient.

Further, the control section 17 monitors the operation of the gas supply system on the basis of the outputs of the flowmeter 7 and the pressure gauge 14. For example, in the case where a predetermined pressure is not detected by the pressure gauge 14 during inspiration, the control section 17 determines that the mask 15 is inappropriately worn. Then, the control section 17 outputs a predetermined notice through a display section 18 or a speaker 19, to thereby call the user's attention. However, the following configuration may be employed: after a predetermined respiratory rate is obtained, the control section 17 closes both of the inspiratory solenoid valve 5 and the expiratory solenoid valve 8, and monitors the output of the pressure gauge 14 at that time, to thereby detect recovery of the spontaneous breathing of the patient.

The display section 18 is constituted of a liquid crystal display, an organic EL display, or the like, and is controlled by the control section 17 to display. The display section 18 displays, according to the output of the control section 17, the condition of the patient and the wearing state of the mask. In addition, the control section 17 controls the display section 18 to display the operating procedure for the artificial respirator 100 (procedure of wearing the mask, resuscitation procedure, and the like). Audio guidance through the speaker 19 may be performed at the same time. In this manner, the first-aid can be carried out by ordinary people other than the healthcare professionals. It should be noted that the display section 18 and the speaker 19 constitute a "guiding means" according to the present invention.

Next, the operation of the artificial respirator 100 according to this embodiment, which is configured in the above-mentioned manner, will be described.

When not used, the inspiratory solenoid valve 5 and the expiratory solenoid valve 8 are, as shown in Fig. 6, in the blocking position and the communication position, respectively. The reservoir tank 4 stores the inspiratory gas of 141.3 kPa in advance. Further, the gas pressure between the pressure reducing valve 6 and the inspiratory solenoid valve 5 is kept at 104.26 kPa.

For use, the mask 15 is put on the patient. The control section 17 controls the inspiratory solenoid valve 5 and the expiratory solenoid valve 8 to be driven, to thereby alternately switch between the supply of the inspiratory gas to the patient and the discharge of the expired air. In this embodiment, according to the respiratory pattern shown in Fig. 1, the inspiratory solenoid valve 5 and the expiratory solenoid valve 8 are driven. The respiratory interval is set to 5 seconds, the inspiration period of time is set to 1.66 seconds, and the expiration period of time is set to 3.33 seconds. That is, the expiration period of time is set to be longer than the inspiration period of time. The control section 17 operates the pressurizing pump 1 at a constant rotational speed (3100 rpm) in a continuous manner at the same time of the start of use.

As described with reference to Fig. 4 and Fig. 5, the pressurizing pump 1 keeps the minimum inspiratory pressure (104.26 kPa), and at the same time supplies the gas amount consumed during expiration before the termination of the expiration period of time. Thus, the gas amount necessary for the inspiration is always ensured in the reservoir tank 4. In particular, according to this embodiment, the expiration period of time is set to be longer than the inspiration period of time, and hence the pressurizing pump 1 can be constituted of a pump having a small volume. Thus, it is possible to achieve a reduction of the size, the weight, and the electric power consumption of the pump. Further, the reservoir tank 4 does not need to have a large volume, and a small tank of about 1 L can be used, which can largely attribute to a reduction of the size and the weight of the apparatus.

Fig. 8 shows an example of the electric power consumption of the pressurizing pump 1, the inspiratory solenoid valve 5, the expiratory solenoid valve 8, the control section 17, the display section 18, the flowmeter 7, the pressure gauge 14, and the solenoid valve 9 for exposure to the atmosphere. The inspiratory solenoid valve 5 and the expiratory solenoid valve 8 are alternately driven, and hence the total value is shown as the electric power consumption. Further, the pressure loss has to be minimized, and hence a case of using relatively large solenoid valves 5, 8, and 9 are illustrated.

In this embodiment, the electric power consumption of the whole of the apparatus is about 62 W. Assumed that the voltage of the battery 20 is 24 V, the electric current value is 2.58 A. In this case, when the battery 20 of 3 AH is used, the operation of 1.1 hours is enabled. The artificial respirator 100 of this embodiment is a respirator for first-aid, and it is sufficient that for example the operation be enabled until the ambulance arrives. Thus, the above-mentioned operation period of time can achieve the object sufficiently.

Although the embodiment of the present invention has been described above, it is needless to say that the present invention is not limited thereto, but various modification can be made on the basis of the technical idea of the present invention.

Although for example in the above-mentioned embodiment, the ratio of the inspiration period of time (first period of time) and the expiration period of time (second period of time) is set to 1:2 so that the expiration period of time is set to be longer than the inspiration period of time, the present invention is not limited thereto, but the expiration period of time may be set to be equal to the inspiration period of time. Further, as long as the expiration period of time is equal to or longer than the inspiration period of time, such a ratio can be appropriately changed. Similarly, the respiratory rate per unit time, the inspiratory gas amount, the minimum inspiratory pressure, and the like can be also changed appropriately. Depending on those conditions, a pump can be appropriately selected as the pressurizing pump.

Further, although in the above-mentioned embodiment, a pair of two-port and two-position solenoid valves 5, 8 are used as the valve mechanism that switches between the expiration and the inspiration, the present invention is not limited thereto. For example, a 3-port and 3-position solenoid valve which has a first position for feeding the inspiratory gas from the reservoir tank to the mask, a second position for blocking between the reservoir tank and the mask, a third position for exposing the mask to the atmosphere may be used as the valve mechanism.

Description of Symbols

| 1 | pressurizing pump |
| 4 | reservoir tank |
| 5 | inspiratory solenoid valve |
| 6 | pressure reducing valve |
| 8 | expiratory solenoid valve |
| 15 | mask |
| 17 | control section |
| 18 | display section |
| 19 | speaker |
| 20 | battery |
| 23 | casing |
| 100 | artificial respirator |

**Claims**

**1.** An artificial respirator, comprising:

a mask that a patient wears;
a reservoir tank that stores inspiratory air;
a valve mechanism that is connected between the mask and the reservoir tank, the valve mechanism having a first state for feeding the inspiratory air contained in the reservoir tank to the mask and a second state for releasing expired air of the patient, which is discharged from the mask;
a control section that is capable of alternately switching between the first state and the second state, and that controls the valve mechanism to be driven so that the first state is kept for a first period of time and the second state is kept for a second period of time equal to or longer than the first period of time; and
a pressurizing pump that is connected to the reservoir tank to supply the inspiratory air, which is consumed in the first period of time, within the second period of time.

2. The artificial respirator according to claim 1, further comprising:

a battery that supplies electric power to the valve mechanism, the control section, and the pressurizing pump; and
a casing that houses the battery, the mask, the reservoir tank, the valve mechanism, the control section, and the pressurizing pump.

3. The artificial respirator according to claim 2, further comprising a guiding means for providing audio or video guidance for an operating procedure for the artificial respirator.

4. An operation method for an artificial respirator, comprising:

feeding inspiratory air within a reservoir tank to a mask, that a patient wears, for a first period of time;
releasing expired air of the patient under a state of blocking a communication between the reservoir tank and the mask for a second period of time equal to or longer than the first period of time;
supplying the inspiratory air, which is consumed in the first period of time, within the second period of time by a pressurizing pump connected to the reservoir tank; and
feeding the inspiratory air contained in the reservoir tank to the mask for the first period of time.

5. The operation method for the artificial respirator according to claim 4, wherein
the pressurizing pump is continuously operated for the first period of time and the second period of time at a constant rotational speed.

FIG.1

FIG.2

FIG.3

FIG.4

FIG.5

FIG.6

FIG.7

| Numeral | Component | Consumed electric power (W) |
|---------|-----------|-----------------------------|
| 1 | Pressurizing pump | 13 |
| 5 | Inspiratory solenoid valve | 14 |
| 8 | Expiratory solenoid valve | ---- |
| 17 | Control system | 10 |
| 18 | Display system | 10 |
| 7 | Flowmeter | 5 |
| 14 | Pressure gauge | 5 |
| 9 | Solenoid valve for exposure to the atmosphere | 5 |
| | Total | 62 |

＊ Solenoid valves 5, 8 are alternately operated

FIG.8

**INTERNATIONAL SEARCH REPORT**

| | International application No. |
|---|---|
| | PCT/JP2010/004858 |

| A. CLASSIFICATION OF SUBJECT MATTER |
|---|
| A61M16/00(2006.01)i |

According to International Patent Classification (IPC) or to both national classification and IPC

| B. FIELDS SEARCHED |
|---|

Minimum documentation searched (classification system followed by classification symbols)
A61M16/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y | JP 2002-540858 A (Event Medical Ltd.), 03 December 2002 (03.12.2002), paragraphs [0009] to [0025]; fig. 1 to 4 & US 6782888 B1 & EP 1171182 A1 & WO 2000/059566 A1 & AU 3448900 A | 1-3 |
| Y | JP 2008-136664 A (Terumo Corp.), 19 June 2008 (19.06.2008), paragraphs [0036] to [0040], [0153] to [0162], [0189]; fig. 1, 7, 24 (Family: none) | 1-3 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 18 August, 2010 (18.08.10) | 31 August, 2010 (31.08.10) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
|---|
| PCT/JP2010/004858 |

C (Continuation). DOCUMENTS CONSIDERED TO BE RELEVANT

| Category* | Citation of document, with indication, where appropriate, of the relevant passages | Relevant to claim No. |
|---|---|---|
| Y | US 2008/0168990 A1  (Richard Henry Cooke), 17 July 2008 (17.07.2008), paragraphs [0053], [0116], [0144] to [0147]; fig. 1 to 5 & JP 2010-506626 A      & US 2007/0221221 A1 & EP 2073879 A2          & WO 2007/101124 A2 | 1-3 |
| A | JP 2005-46408 A  (Teijin Ltd.), 24 February 2005 (24.02.2005), paragraphs [0032], [0037]; fig. 1 to 2 (Family: none) | 1-3 |
| A | JP 9-182793 A  (Teijin Ltd.), 15 July 1997 (15.07.1997), entire text; all drawings (Family: none) | 1-3 |
| A | JP 61-131756 A  (Toru SATO), 19 June 1986 (19.06.1986), entire text; all drawings & US 4681099 A            & EP 0188071 A1 | 1-3 |

Form PCT/ISA/210 (continuation of second sheet) (July 2009)

**INTERNATIONAL SEARCH REPORT**

| International application No. |
| --- |
| PCT/JP2010/004858 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 4 - 5
    because they relate to subject matter not required to be searched by this Authority, namely:
    Claims 4 and 5 is concerned with method for operation of an artificial aspirator which may include the acts by a doctor or the like and pertain to method for treatment of the human body by therapy.
    (continued to extra sheet)

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2010/004858

Continuation of Box No.II-1 of continuation of first sheet(2)

Therefore, claims 4 and 5 relate to a subject on which this International Searching Authority is not required to carry out an international search under the provisions of PCT Article 17(2)(a)(i) and PCT Rule 39.1(iv).

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007525273 A